# EUROPEAN PATENT APPLICATION

(11) **EP 4 848 307 A1**
(43) Date of publication of application: **02.12.2026**
(21) Application number: 26181075.8
(22) Date of filing: 26.05.2026
(51) Int. Cl.: G16H 50/30, G16H 50/20, G16H 10/60, G16H 50/70

(54) **METHODS AND APPARATUS FOR DETERMINING AND/OR PREDICTING CARDIOGENIC SHOCK SEVERITY**

(30) Priority: 28.05.2025 US 202563813128 P
(71) Applicant: Abiomed, Inc., Danvers, MA 01923 (US)
(72) Inventor: MOYER, Christian, Danvers, 01923 (US); BALK, Steven A., Danvers, 01923 (US)
(74) Representative: Wittmer, Maximilian

(57) **Abstract**

Methods and apparatus for determining cardiogenic shock information for a patient using a medical device are described. The method includes receiving, from an electronic medical record associated with a patient, medical information for the patient, extracting values for a set of features associated with cardiogenic shock from the received medical information, receiving device information associated with operation of the medical device, determining based, at least in part, on the values for the set of features and the device information, cardiogenic shock information for the patient, wherein the cardiogenic shock information includes a severity of cardiogenic shock for the patient, and outputting on a user interface associated with the medical device, an indication of the cardiogenic shock information.

## Description

### FIELD OF THE INVENTION

This disclosure relates to techniques for determining and/or predicting cardiogenic shock severity for a patient.

### BACKGROUND

Cardiogenic shock is a life threatening medical emergency where a patient's heart cannot pump enough blood to meet the body's needs. The Society for Cardiovascular Angiography & Interventions (SCAI) has released a classification of cardiogenic shock which ranges from stage A, "at risk" to E, "extremis." The SCAI classification has been endorsed and is generally accepted for classifying cardiogenic shock stage. The SCAI classification profiles the patient's stage based on a clinical assessment, hemodynamic parameters, metabolic derangements, echocardiographic findings, and pulmonary artery catheter monitoring.

### SUMMARY

Described herein are systems and methods for predicting current and/or future cardiogenic shock severity for a patient based on medical information for the patient and information about operation of a medical device (e.g., a heart pump) associated with the patient. Some conventional techniques for determining cardiogenic shock severity may not take into consideration whether a patient is using a medical device and/or how the medical device is being used (e.g., the level of cardiac support the medical device is providing). Some embodiments of the present disclosure take information about operation of a medical device associated with a patient into account when determining a cardiogenic shock severity, which may more accurately reflect the underlying cardiac condition of the patient compared with techniques that do not consider such information. For instance, patient information received from a medical information system and/or medical device operation data (e.g., pump speed for a heart pump) may be used in some embodiments to determine a severity of cardiogenic shock for a patient as if the patient was not using the medical device. The severity information may be used by a medical provider to, for example, determine when and/or how to wean the patient off of the medical device and/or control operation of the medical device to provide additional support to the patient, if needed. In some embodiments, the patient information and/or the medical device operation information may be used to predict a future cardiogenic shock severity for the patient, which may help inform medical treatment provided by a healthcare provider and/or reduce a risk that the patient experiences severe cardiogenic shock.

In some embodiments, a system of determining cardiogenic shock information for a patient using a medical device is provided. The system comprises a a controller in communication with a medical information system and the medical device. The controller is configured to execute a method. The method includes receiving, from an electronic medical record associated with a patient (i.e., from the medical information system), medical information for the patient, extracting values for a set of features associated with cardiogenic shock from the received medical information, receiving device information associated with operation of the medical device, determining based, at least in part, on the values for the set of features and the device information, cardiogenic shock information for the patient, wherein the cardiogenic shock information includes a severity of cardiogenic shock for the patient, and outputting on a user interface associated with the medical device, an indication of the cardiogenic shock information.

In one aspect, the medical information for the patient includes one or more of an electronic health record, patient physiological information, patient laboratory data, or physician notes. In another aspect, the medical device is at least one of a mechanical circulatory support device or an extracorporeal membrane oxygenation (ECMO) machine. In another aspect, the device information associated with operation of the medical device includes one or more of medical device information for the medical device, operating conditions of the medical device, and medical device flow rate. In another aspect, the set of features includes one or more of blood pressure, blood flow, lactate level, blood pH level, or medication rate.

In another aspect, determining cardiogenic shock information for the patient by the controller comprises providing the values of the set of features and the device information as input to a trained machine learning model configured to determine the cardiogenic shock information. In another aspect, determining cardiogenic shock information for the patient by the controller comprises determining a classification of severity of cardiogenic shock for the patient. In another aspect, the classification includes an alphabetic value associated with the severity of cardiogenic shock for the patient, and outputting on a user interface an indication of the cardiogenic shock information comprises displaying the alphabetic value and/or information based on the alphabetic value. In another aspect, the classification is along a continuous scale including numerical values associated with the severity of cardiogenic shock for the patient, and outputting on the user interface an indication of the cardiogenic shock information comprises displaying a numerical value of the cardiogenic shock information and/or information based on the numerical value. In another aspect, the classification is continuously determined. In another aspect, the classification is determined at a set interval.

In another aspect, determining cardiogenic shock information for the patient by the controller further includes providing the values for the set of features and the device information to a decision tree architecture configured to determine the cardiogenic shock information. In another aspect, the method further includes determining that at least one value in the set of features from the received medical information is missing or invalid. In another aspect, the method further includes determining a replacement value for the at least one feature value that is not present in the set of features or is invalid, wherein determining cardiogenic shock information is based on the set of features including the replacement value. In another aspect, the value in the set of features is determined to be invalid based at least in part on the value being outside of an expected range or a time associated with the value is greater than a threshold amount of time relative to a present time. In another aspect, the replacement value is an estimate of the value for the invalid or missing feature based on an average of a plurality of patients. In another aspect the method further includes outputting an indication based on the at least one value in the set of features being missing or invalid. In another aspect, outputting the indication based on the at least one value in the set of features missing or being invalid comprises displaying on a user interface a color coded indication associated with the missing or invalid value. In another aspect, outputting the indication based on the at least one value in the set of features missing or being invalid by the controller comprises displaying on the user interface a recommendation to perform one or more tests on the patient based on the missing or invalid value in the set of features. In another aspect, determining cardiogenic shock information for the patient by the controller includes determining a weighted value for each of the values in the set of features, and determining a weighted feature value for the set of features based on the weighted value and the values in the set of features, wherein determining cardiogenic shock information is based at least in part on the weighted feature values for the set of features and the device information. In another aspect, the weighted value is based, at least in part, on the whether the value is an invalid value, and the weighted value for the invalid values is less than the weighted value for valid values of the set of features.

In another aspect, the method further includes performing based on the cardiogenic shock information, categorization of the patient into a risk group of a plurality of risk groups, wherein outputting the indication of the cardiogenic shock information comprises outputting an indication of the risk group for the patient. In another aspect, the risk group is associated with a future severity of cardiogenic shock for the patient. In another aspect, outputting an indication of the risk group for the patient by the controller comprises displaying on a user interface a color coded indication of the risk group. In another aspect, the controller is further configure to receive a user input via the user interface to change one or more values associated with the device information, to simulate a cardiogenic shock assessment to predict the severity of the cardiogenic shock of the patient based, at least in part, on the changed one or more values, to generate simulated cardiogenic shock information, and displaying, on the user interface, a simulated indication based at least in part on the simulated cardiogenic shock information. In another aspect, the medical device is a mechanical circulatory support device, and the one or more values associated with the device information includes one or more of a flow rate, a pump speed and a pressure signal associated with the mechanical circulatory support device.

In another aspect, the medical device is a mechanical circulatory support device, and the received device information includes at least one of a pump speed or a pressure signal associated with the mechanical circulatory support device. In another aspect, the cardiogenic shock information indicates a cardiogenic shock stage of the patient without the medical device. In another aspect, determining cardiogenic shock information for the patient comprises determining a weighted value for each of the values in the set of features, determining a weighted feature value for the set of features based on the weighted value and the values for the set of features, and determining, based at least in part on the weighted feature values for the set of features and the device information, cardiogenic shock information.

In some embodiments, a system of predicting a future severity of cardiogenic shock for a patient using a medical device is provided. The system comprises a controller in communication with a medical information system and the medical device, the controller being configured to execute a method. The method includes receiving, from an electronic medical record associated with a patient (i.e. from the medical information system), medical information for the patient, extracting values for a set of features associated with cardiogenic shock from the received medical information, receiving device information associated with operation of the medical device, determining based, at least in part, on the values for the set of features and the device information, cardiogenic shock information for the patient, wherein the cardiogenic shock information includes a future severity of cardiogenic shock for the patient, and outputting on a user interface associated with the medical device, an indication of the cardiogenic shock information.

In some embodiments, the system comprises the medical device.

In some embodiments, a method of determining cardiogenic shock information for a patient using a medical device is provided. The method includes receiving, from an electronic medical record associated with a patient, medical information for the patient, extracting values for a set of features associated with cardiogenic shock from the received medical information, receiving device information associated with operation of the medical device, determining based, at least in part, on the values for the set of features and the device information, cardiogenic shock information for the patient, wherein the cardiogenic shock information includes a severity of cardiogenic shock for the patient, and outputting on a user interface associated with the medical device, an indication of the cardiogenic shock information.

In one aspect, the medical information for the patient includes one or more of an electronic health record, patient physiological information, patient laboratory data, or physician notes. In another aspect, the medical device is at least one of a mechanical circulatory support device or an extracorporeal membrane oxygenation (ECMO) machine. In another aspect, the device information associated with operation of the medical device includes one or more of medical device information for the medical device, operating conditions of the medical device, and medical device flow rate. In another aspect, the set of features includes one or more of blood pressure, blood flow, lactate level, blood pH level, or medication rate.

In another aspect, determining cardiogenic shock information for the patient comprises providing the values of the set of features and the device information as input to a trained machine learning model configured to determine the cardiogenic shock information. In another aspect, determining cardiogenic shock information for the patient comprises determining a classification of severity of cardiogenic shock for the patient. In another aspect, the classification includes an alphabetic value associated with the severity of cardiogenic shock for the patient, and outputting on a user interface an indication of the cardiogenic shock information comprises displaying the alphabetic value and/or information based on the alphabetic value. In another aspect, the classification is along a continuous scale including numerical values associated with the severity of cardiogenic shock for the patient, and outputting on the user interface an indication of the cardiogenic shock information comprises displaying a numerical value of the cardiogenic shock information and/or information based on the numerical value. In another aspect, the classification is continuously determined. In another aspect, the classification is determined at a set interval.

In another aspect, determining cardiogenic shock information for the patient further includes providing the values for the set of features and the device information to a decision tree architecture configured to determine the cardiogenic shock information. In another aspect, the method further includes determining that at least one value in the set of features from the received medical information is missing or invalid. In another aspect, the method further includes determining a replacement value for the at least one feature value that is not present in the set of features or is invalid, wherein determining cardiogenic shock information is based on the set of features including the replacement value. In another aspect, the value in the set of features is determined to be invalid based at least in part on the value being outside of an expected range or a time associated with the value is greater than a threshold amount of time relative to a present time. In another aspect, the replacement value is an estimate of the value for the invalid or missing feature based on an average of a plurality of patients. In another aspect the method further includes outputting an indication based on the at least one value in the set of features being missing or invalid. In another aspect, outputting the indication based on the at least one value in the set of features missing or being invalid comprises displaying on a user interface a color coded indication associated with the missing or invalid value. In another aspect, outputting the indication based on the at least one value in the set of features missing or being invalid comprises displaying on the user interface a recommendation to perform one or more tests on the patient based on the missing or invalid value in the set of features. In another aspect, determining cardiogenic shock information for the patient includes determining a weighted value for each of the values in the set of features, and determining a weighted feature value for the set of features based on the weighted value and the values in the set of features, wherein determining cardiogenic shock information is based at least in part on the weighted feature values for the set of features and the device information. In another aspect, the weighted value is based, at least in part, on the whether the value is an invalid value, and the weighted value for the invalid values is less than the weighted value for valid values of the set of features.

In another aspect, the method further includes performing based on the cardiogenic shock information, categorization of the patient into a risk group of a plurality of risk groups, wherein outputting the indication of the cardiogenic shock information comprises outputting an indication of the risk group for the patient. In another aspect, the risk group is associated with a future severity of cardiogenic shock for the patient. In another aspect, outputting an indication of the risk group for the patient comprises displaying on a user interface a color coded indication of the risk group. In another aspect, the method further includes receiving a user input via the user interface to change one or more values associated with the device information, simulating a cardiogenic shock assessment to predict the severity of the cardiogenic shock of the patient based, at least in part, on the changed one or more values, to generate simulated cardiogenic shock information, and displaying, on the user interface, a simulated indication based at least in part on the simulated cardiogenic shock information. In another aspect, the medical device is a mechanical circulatory support device, and the one or more values associated with the device information includes one or more of a flow rate, a pump speed and a pressure signal associated with the mechanical circulatory support device.

In another aspect, the medical device is a mechanical circulatory support device, and the received device information includes at least one of a pump speed or a pressure signal associated with the mechanical circulatory support device. In another aspect, the cardiogenic shock information indicates a cardiogenic shock stage of the patient without the medical device. In another aspect, determining cardiogenic shock information for the patient comprises determining a weighted value for each of the values in the set of features, determining a weighted feature value for the set of features based on the weighted value and the values for the set of features, and determining, based at least in part on the weighted feature values for the set of features and the device information, cardiogenic shock information.

In some embodiments, a method of predicting a future severity of cardiogenic shock for a patient using a medical device is provided. The method includes receiving, from an electronic medical record associated with a patient, medical information for the patient, extracting values for a set of features associated with cardiogenic shock from the received medical information, receiving device information associated with operation of the medical device, determining based, at least in part, on the values for the set of features and the device information, cardiogenic shock information for the patient, wherein the cardiogenic shock information includes a future severity of cardiogenic shock for the patient, and outputting on a user interface associated with the medical device, an indication of the cardiogenic shock information.

### BRIEF DESCRIPTION OF DRAWINGS

FIG. 1 shows an illustrative mechanical circulatory support system, in accordance with some embodiments.
FIG. 2 illustrates a block diagram of a system architecture, in accordance with some embodiments.
FIG. 3 illustrates a flowchart of a process for predicting cardiogenic shock severity information for a patient, in accordance with some embodiments.
FIG. 4 illustrates a flowchart of a process for replacing invalid and/or missing values in medical information for a patient, which may be used to determine cardiogenic shock severity information for a patient, in accordance with some embodiments.
FIG. 5 illustrates a flowchart of a process for performing a simulation to simulate how cardiogenic shock severity predictions are impacted by different medical interventions provided to the patient, in accordance with some embodiments.

### DETAILED DESCRIPTION

Some conventional techniques for assessing cardiogenic shock severity do not take into consideration the presence of medical devices (e.g., an implanted heart pump) that may impact the cardiogenic shock score for the patient, which may artificially present the patient as having a less severe cardiogenic shock classification than their underlying cardiac health indicates. Some embodiments leverage medical information about the patient and/or operation information for a medical device associated with the patient to improve the process of cardiogenic shock severity determination and/or prediction. Such an improved cardiogenic shock severity determination and/or prediction process may enable a healthcare provider (e.g., a physician) to better understand a patient's cardiac health state. A better understanding of the patient's cardiac health state (e.g., current or future state) may inform patient care such that the healthcare provider may provide appropriate preemptive cardiac support (e.g., use of a mechanical circulatory support device and/or changing one or more operating conditions of a medical device being used by the patient) to reduce the risk that the patient will enter a state of severe cardiogenic shock. Such preemptive support may provide the patient with a decreased chance of cardiac complications and/or an increased chance of survival following surgery compared with conventional reactive approaches where preemptive cardiac support is not provided or altered until the patient shows evidence of a worsened cardiogenic shock severity.

To provide an overall understanding of the systems, methods, and devices described herein, certain illustrative examples will be described. Although various examples may describe specific medical procedures and/or uses of a cardiac support device, it will be understood that the technology described herein may be employed in any suitable context.

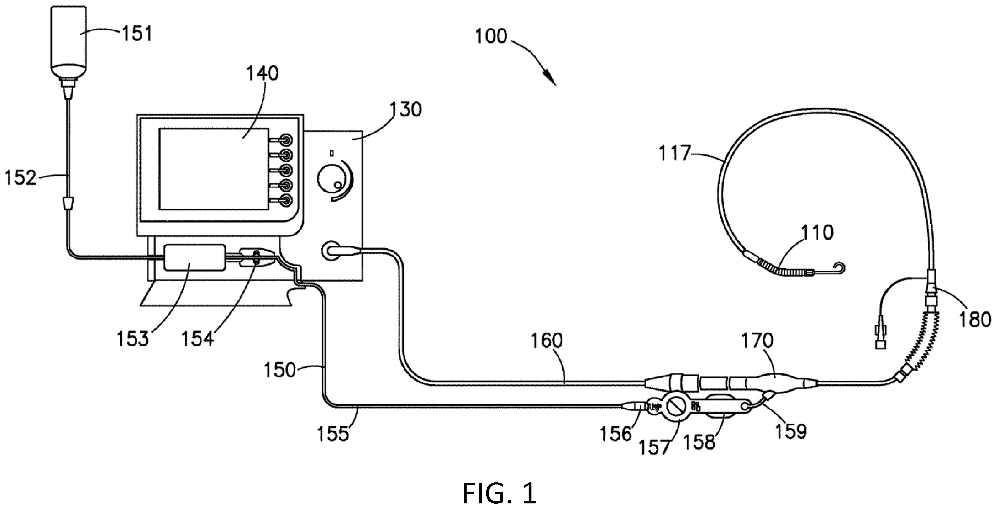

A circulatory support device (also referred to herein as a "heart pump" or simply a "pump") may include a percutaneous, catheter-based device that provides hemodynamic support to the heart of a patient. As shown in FIG. 1, heart pump 110 may form part of a cardiac support system 100. Cardiac support system 100 also may include a controller 130 (e.g., an Automated Impella Controller^{®}, referred to herein as an "AIC," from ABIOMED, Inc., Danvers, Mass.), a display 140, a purge subsystem 150, a connector cable 160, a plug 170, and a repositioning unit 180. As shown, controller 130 may include display 140. Controller 130 may be configured to monitor and control operation of heart pump 110. During operation, purge subsystem 150 may be configured to deliver a purge fluid to heart pump 110 through catheter tube 117 to prevent blood from entering the motor (not shown) of the heart pump. In some implementations, the purge fluid is a dextrose solution (e.g., 5% dextrose in water with 25 or 50 IU/mL of heparin, although the solution need not include heparin in all embodiments). Connector cable 160 may provide an electrical connection between heart pump 110 and controller 130. Plug 170 may connect catheter tube 117, purge subsystem 150, and connector cable 160. In some implementations, plug 170 may include a storage device (e.g., a memory) configured to store, for example, operating parameters to facilitate transfer of the patient to another controller if needed. Repositioning unit 180 may be used to reposition heart pump 110 in the patient's heart.

As shown in FIG. 1, in some embodiments, the cardiac support system 100 may include a purge subsystem 150 having a container 151, a supply line 152, a purge cassette 153, a purge disc 154, purge tubing 155, a check valve 156, a pressure reservoir 157, an infusion filter 158, and a sidearm 159. Container 151 may, for example, be a bag or a bottle. As will be appreciated, in other embodiments the cardiac support system 100 may not include a purge subsystem. In some embodiments, a purge fluid may be stored in container 151. Supply line 152 may provide a fluidic connection between container 151 and purge cassette 153. Purge cassette 153 may control how the purge fluid in container 151 is delivered to heart pump 110. For example, purge cassette 153 may include one or more valves for controlling a pressure and/or flow rate of the purge fluid. Purge disc 154 may include one or more pressure and/or flow sensors for measuring a pressure and/or flow rate of the purge fluid. As shown, controller 130 may include purge cassette 153 and purge disc 154. Purge tubing 155 may provide a fluidic connection between purge disc 154 and check valve 156. Pressure reservoir 157 may provide additional filling volume during a purge fluid change. In some implementations, pressure reservoir 157 includes a flexible rubber diaphragm that provides the additional filling volume by means of an expansion chamber. Infusion filter 158 may help prevent bacterial contamination and air from entering catheter tube 117. Sidearm 159 may provide a fluidic connection between infusion filter 158 and plug 170. Although shown as having separate purge tubing and connector cable, it will be appreciated that in some embodiments, the cardiac support system 100 may include a single connector with both fluidic and electric lines connectable to the controller 130.

During operation, controller 130 may be configured to receive measurements from one or more pressure sensors (not shown) included as a portion of heart pump 110 and purge disc 154. Controller 130 may also be configured to control operation of the motor (not shown) of the heart pump 110 and purge cassette 153. As noted herein, controller 130 may be configured to control and measure a pressure and/or flow rate of a purge fluid via purge cassette 153 and purge disc 154. During operation, after exiting purge subsystem 150 through sidearm 159, the purge fluid may be channeled through purge lumens (not shown) within catheter tube 117 and plug 170. Sensor cables (not shown) within catheter tube 117, connector cable 160, and plug 170 may provide an electrical connection between components of the heart pump 110 (e.g., one or more pressure sensors) and controller 130. Motor cables (not shown) within catheter tube 117, connector cable 160, and plug 170 may provide an electrical connection between the motor of the heart pump 110 and controller 130. During operation, controller 130 may be configured to receive measurements from one or more pressure sensors of the heart pump 110 through the sensor cables (e.g., optical fibers) and to control the electrical power delivered to the motor of the heart pump 110 through the motor cables. By controlling the power delivered to the motor of the heart pump 110, controller 130 may be operable to control the speed of the motor.

Various modifications can be made to cardiac support system 100 and one or more of its components. For instance, one or more additional sensors may be added to heart pump 100. In another example, a signal generator may be added to heart pump 100 to generate a signal indicative of the rotational speed of the motor of the heart pump 110. As another example, one or more components of cardiac support system 100 may be separated. For instance, display 140 may be incorporated into another device in communication with controller 130 (e.g., wirelessly or through one or more electrical cables).

A heart pump (e.g., heart pump 110) may include a pressure sensor (e.g., an optical pressure sensor) configured to detect a pressure within the aorta of a patient's heart when the heart pump is properly positioned in the left side of the heart. The pressure signal sensed by the pressure sensor may be used, at least in part, to determine correct positioning of the heart pump within the patient's heart and/or to determine a blood flow rate through the heart pump when in operation. For instance, the pressure signal may be used in combination with a motor current signal received from a motor current sensor (not shown) and a set of stored values to determine a flow rate of blood through the heart pump. The differential pressure across the aortic valve may also indirectly be determined based on the pressure signal measuring the pressure in the aorta and the set of stored values.

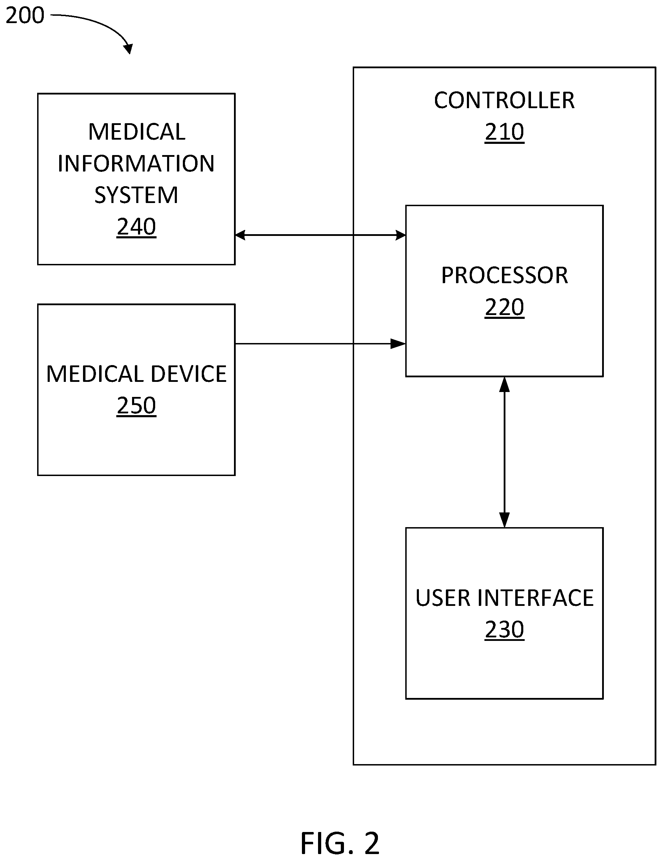

FIG. 2 schematically illustrates an example system 200, in accordance with some embodiments of the present disclosure. The system 200 may include a controller 210 in communication with a medical information system 240 and a medical device 250. It should be appreciated that any of the components shown in system 200 may be coupled wirelessly and/or using one or more wired connections. The medical information system 240 may include, for example, a storage device configured to store one or more electronic health records, a medical facility laboratory database, a medical facility radiology database or any other suitable data storage configured to store patient related health information. The medical device 250 may include, for example, a mechanical circulatory support device, such as an intravascular blood pump, a heart pump (e.g., the heart pump 110), or an intracardiac pump device, an extracorporeal membrane oxygenation (ECMO) machine, or any other suitable medical device.

The controller 210 may include a processor 220 and a user interface 230. In some embodiments, the user interface 230 may be integrated with the controller 210. For example, the user interface 230 may be implemented as a touch interface (e.g., touchscreen) on the controller 210 configured to display indications to a healthcare provider and/or receive inputs from a user. In the example system 200 shown in FIG. 2, user interface 230 is shown as being integrated with or otherwise included as a portion of controller 210. It should be appreciated, however, that user interface 230 may be implemented in some embodiments as a component separate from controller 210. For instance, a computing device associated with the patient may be configured to provide user interface 230. The computing device may be a mobile device, tablet and/or a computer system. The user interface 230 may be configured to receive inputs from a user. For example, the user interface 230 may include a touchscreen or input device (e.g., keyboard) associated with the controller 210 configured to receive inputs from the user. In the example system 200 shown in FIG. 2, processor 220 is shown as being integrated with or otherwise included as a portion of controller 210. It should be appreciated, however, that processor 220 may be implemented in some embodiments as a component separate from controller 210. For instance, processor 220 may be implemented as a portion of a networked (e.g., cloud-based) computing device.

In some embodiments, the processor 220 and/ or the controller 210 may be configured to extract values for one or more features from medical information received from medical information system 240. The medical information may include any suitable information associated with the patient including, but not limited to, patient health information and/or patient demographic information. For example, the medical information may include, but is not limited to, one or more of an electronic health record of the patient, physiological information associated with the patient, laboratory test data associated with the patient, and physician notes associated with the patient. The one or more features extracted from the medical information may include, but are not limited to, blood pressure, blood flow, lactate level, blood pH level and a rate of medication received by the patient.

In some embodiments, the processor 220 and/ or the controller 210 may be configured to receive device information associated with operation of the medical device 250 being used by a patient. The device information may include, but is not limited to, the type of medical device 250 and one or more operating conditions of the medical device 250. The device information received by processor 220 may depend, at least in part, on the type of medical device 250. For example, the device information for a mechanical circulatory support device may include, but is not limited to, one or more of flow rate, a pump speed, and a pressure signal associated with the mechanical circulatory support device. In another example, the device information for an ECMO machine may include, but is not limited to, one or more of blood oxygenation, pump flow rate, a pump speed, gas flow and a pressure signal associated with the ECMO machine.

In some embodiments, the processor 220 and/ or the controller 210 may be configured to determine cardiogenic shock severity information for the patient based, at least in part, on the values of the one or more features extracted from the medical information and/or the device information. The cardiogenic shock severity information may indicate a severity of cardiogenic shock for the patient with the medical device 250. In some embodiments, the cardiogenic shock severity information may be associated with a discrete category of a plurality of categories, for example, the SCAI classifications (e.g., "A," "B," "C," etc.). In some embodiments, the cardiogenic shock severity information may be determined along a continuous scale, such as using numerical values (e.g., 0-100). It should be appreciated that in some embodiments, the cardiogenic shock severity information may be a category determined based on a range of numerical values.

In some embodiments, the processor 220 and/ or the controller 210 may be configured to output an indication of the cardiogenic shock severity information on the user interface 230. For example, the indication provided on the user interface 230 may be an alphanumeric value, which may enable a healthcare provider to better understand a patient's current cardiac health status. In some embodiments, the processor 220 and/ or the controller 210 may be configured to output the indication of the cardiogenic shock severity information to the medical information system 240, where the cardiogenic shock severity information (or information based on the cardiogenic shock severity information) may be stored and/or added to an electronic health record for the patient.

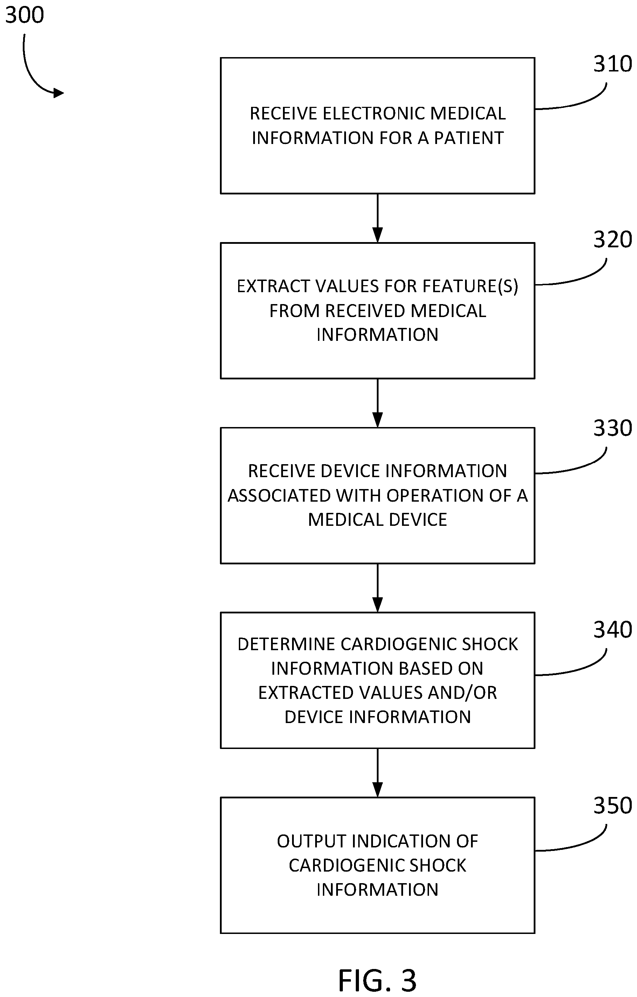

FIG. 3 shows a flowchart of a process 300 for determining cardiogenic shock severity information, in accordance with some embodiments of the present disclosure. In act 310, electronic medical information associated with a patient may be received. For instance, as described above, an electronic health record, laboratory test data, or any other suitable electronic medical information may be received from a medical information system (e.g., medical information system 240). In some embodiments, at least some of the electronic medical information may be received automatically from the medical information system. In some embodiments, at least some of the electronic medical information may be received via a user interface (e.g., user interface 230) configured to receive input from a healthcare provider or other user.

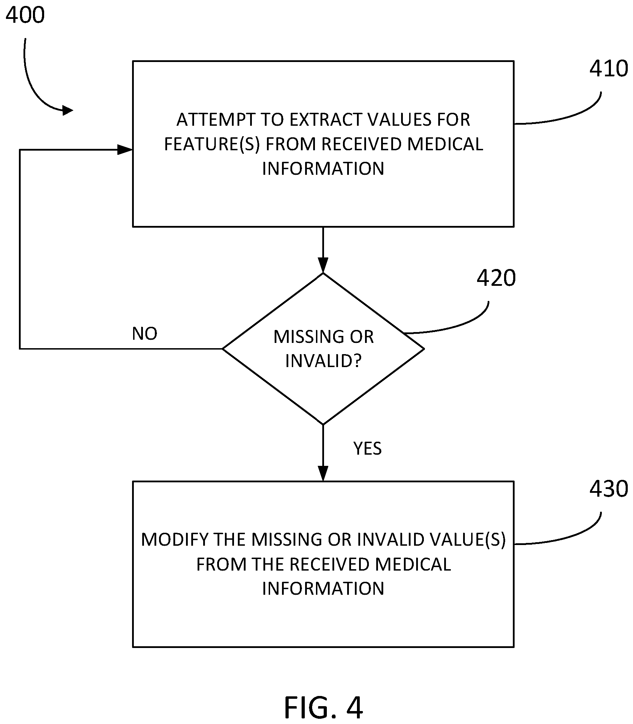

Process 300 may then proceed to act 320, where values for one or more features are extracted from the received medical information. The one or more features may include, but are not limited to, the features described above. In some embodiments, a set of features used to determine cardiogenic shock severity information may be defined, and values for each of the features in the set of features may be extracted from the received medical information. In some instances, values for one or more of the features in the set of features may be missing from the received medical information and/or may be invalid (e.g., because the values are old or out of a typical range). An example process of accounting for missing or invalid values for one or more features used to determine cardiogenic shock severity information is described herein in connection with FIG. 4.

Process 300 may then proceed to act 330, where device information associated with a medical device (e.g., the medical device 250) may be received. The device information may include, but is not limited to, information describing a type of the medical device, information describing an operating state of the medical device, or information describing a configuration of the medical device.

Process 300 may then proceed to act 340, where cardiogenic shock severity information (e.g., a current cardiogenic shock severity or a future cardiogenic shock severity) is determined based, at least in part, on the extracted values of the one or more features and/or the received device information. The extracted feature value(s) and device information may be provided, for example, as input to a model (e.g., a trained machine learning model, a decision tree architecture, etc.) trained or otherwise configured to output the cardiogenic shock severity information (also referred to herein as a "cardiogenic shock severity"). Alternatively, the extracted feature value(s) and device information may be provided as input to an algorithm that weights the values of the features to determine a cardiogenic shock severity for the patient. In some embodiments, values for two or more features may be combined to create a derived feature that is not present in the received medical information or received device information. For instance, the values for two or more features may be added, multiplied, subtracted, divided or combined in any other way to generate a value for the derived feature. Values for such derived features may be used to train a machine learning model and/or may be provided as input to the trained machine learning model in accordance with some embodiments. Examples of trained machine learning models include, but are not limited to, a neural network (e.g., a deep neural network), a logistic regression model, a random forest model, a Naïve Bayesian model, and a decision tree model. Any single type of model or combinations of multiple types of models may be used in accordance with the techniques described herein. In some embodiments, the trained machine learning model(s) may use ensemble methods, regression methods, or any other suitable methods for identifying the features and/or combinations of features that are most predictive (e.g., by associating them with greater weights) of the current cardiogenic shock severity and/or a future cardiogenic shock severity of a patient.

In some embodiments, the values associated with each of the feature(s) and device information may be weighted evenly. In other embodiments, the data associated with each of the feature(s) and device information may be weighted differently.

In some embodiments, the cardiogenic shock severity information determined in act 340 may be represented as one of a plurality of risk categories (e.g., high-risk, medium-risk, low-risk) indicating an extent to which the patient is at future risk of a deteriorated cardiogenic shock severity. Additionally, or alternatively, the risk categories may indicate the severity of the cardiogenic shock stage of the patient at the time the values of the features were recorded.

Process 300 may then proceed to act 350, where an indication of the cardiogenic shock severity information determined in act 340 may be output. The indication of the cardiogenic shock severity information may be output in any suitable format. For instance, the indication of the cardiogenic shock severity information may include a numerical value on a continuous scale (e.g., 0-100) and/or a range of numerical values that represents a severity of cardiogenic shock for the patient. Outputting the indication of the cardiogenic shock severity information may include displaying the numerical value and/or a range of numerical values on a user interface (e.g., the user interface 230) of a computing device. In embodiments where the cardiogenic shock severity information is represented by one of a plurality of risk categories, the risk category may be output in any suitable way. For instance, a visual indicator (e.g., red, yellow, green) may be displayed (e.g., on the user interface 230, in the patient's electronic health record, etc.) to indicate the current cardiogenic shock severity of the patient or a prediction of a future cardiogenic shock severity of the patient. In some embodiments, both a visual indicator (e.g., red, yellow, green) and a numerical value, numerical range, percentage, or alphabetic value may be output in act 350 to indicate the current cardiogenic shock severity and/or a prediction of a future cardiogenic shock severity of the patient. In some embodiments, process 300 may be performed for each of a plurality of patients in a medical facility, and a list of patients and their associated cardiogenic shock severities may be output in act 350.

As will be appreciated, process 300 may be repeated for the same patient over a desired period of time, such as while the patient is an inpatient at a medical facility. In such instances, the output at act 350 may change depending upon updated received medical information (received at act 310) and/or updated device information (received at act 330) used to determine the cardiogenic shock severity information at act 340. By updating the cardiogenic shoch severity information over a period of time, a healthcare provider may proceed to treat the patient based on the current view and/or predicted view of the patient's cardiac health. For example, based on the output in act 350, the healthcare provider may determine whether to and/or when to wean a patient off the medical device.

In some embodiments, patient cardiogenic shock severity information may be recalculated after additional information is made available. The additional information may include, but is not limited to, additional medical information for the patient, medical information for a plurality of patients at the medical facility at which surgery was performed on the patient, or additional device information for the medical device associated with the patient. In some embodiments, the cardiogenic shock severity information may be updated after such additional information is made available, with an updated indication of the new cardiogenic shock severity information being output in act 350.

The indication output in act 350 may be provided to a physician and/or other healthcare provider in any suitable way. For instance, the healthcare provider may be provided with information on the user interface that provides an indication of the output of a risk model used to determine the cardiogenic shock severity information and/or describes how the output was determined based on the values of the extracted features and/or device information.

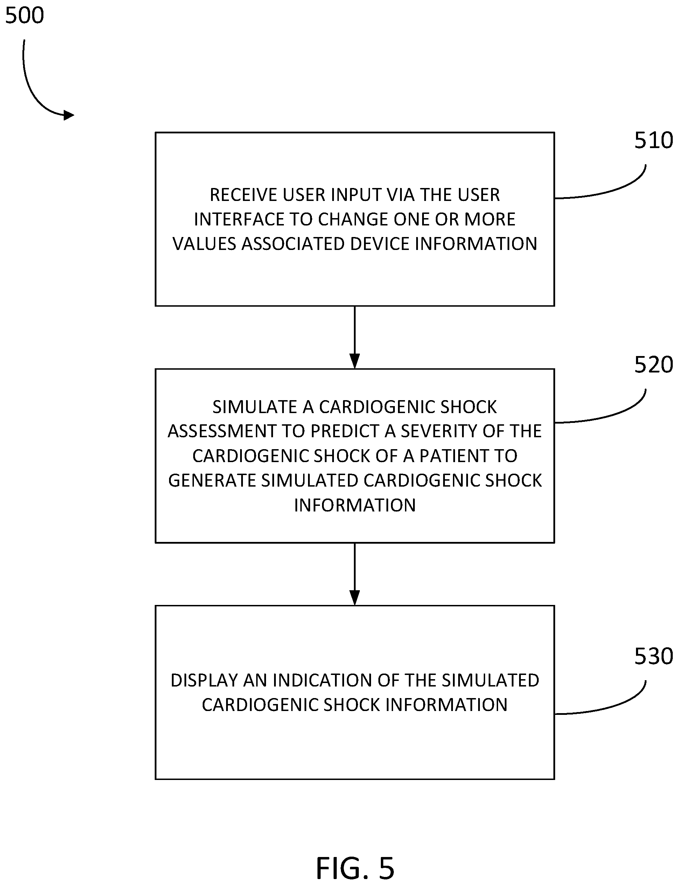

The inventors have recognized and appreciated that a healthcare provider may benefit from simulating how changes in one or more values received in acts 310 and/or 330 change the determination of the cardiogenic shock severity information for a patient. For instance, some embodiments enable a healthcare provider to simulate how providing a treatment to a patient (e.g., by increasing or lowering one or more medical information values, by changing operation of a medical device the patient is using, etc.) would result in a change to the cardiogenic shock severity information output in act 350. Simulating one or more changes in the patient's medical information or device information may provide the healthcare provider with, for example, better insights into when to wean the patient off of medical device support or increase medical device support. In some embodiments, a user (e.g., the healthcare provider) may interact with a user interface (e.g., user interface 230) to change one or more values used to determine cardiogenic shock severity information. For example, the healthcare provider may change one or more operating conditions of a medical device the patient is using to see if the change in operating conditions results in a change to the determined cardiogenic shock severity information (e.g., changes the current cardiogenic shock severity or the future cardiogenic shock severity) as shown connection with FIG. 5. In some embodiments, the information about the cardiogenic shock severity output in act 350 may prompt, suggest, or recommend one or more tests be performed on the patient to improve the accuracy of the cardiogenic shock severity information output in act 350.

FIG. 4 shows a flowchart of process 400 for addressing the existence of one or more invalid or missing values for feature(s) used to determine cardiogenic shock severity information for a patient, in accordance with some embodiments of the present disclosure. In act 410, an attempt is made to extract values for one or more features from received medical information. For example, a set of features used to determine cardiogenic shock severity information for a patient may be defined in advance of determining the cardiogenic shock severity information, and a processor and/ or the controller may be configured to attempt to extract values for each of the features included in the set of features.

Process 400 may then proceed to act 420, where it is determined whether one or more of the values for the features in the set of features is missing from the medical information or is invalid. In some embodiments, a value may be missing if a data field for the feature is empty. For example, the set of features may include a feature of lactate level. However, if a test for determining a patient's lactate level has not yet been performed, the received medical information may not include a value for lactate level and it may be determined in act 420 that the value for lactate level is missing. As another example, a test for determining the patient's lactate level may have been performed, but the lactate level value may not accurately represent the patient's current lactate level. For instance, the test may have been performed too long ago and the data value in the electronic medical information may be stale. In such an instance, the value for lactate level may be considered invalid. Determining whether a value for a feature is stale, and therefore invalid, may be based in part on the feature being considered. For example, values for certain features (e.g., a patient's height or weight) may need to be updated infrequently to not be considered stale, whereas other certain features (e.g., blood glucose values) may need to be updated more frequently to be considered current and not stale. It should be appreciated that a value for a feature may be considered invalid for reasons other than being stale. For example, a value may be considered invalid if the value is not within an expected or typical range of values for the feature, which may indicated, for example, that the value was entered incorrectly into the patient's electronic health record. As an example, a value for a particular feature may only be considered valid when the extracted value is within a certain percentage of a prior value or an expected value for the feature. As another example, a value for a feature may be considered invalid if the value associated with the feature is present in the received medical information, but is unable to be extracted.

If it is determined in act 420 that a value for a feature is not missing or invalid, process 400 may return to act 410 where one or more values for one or more other features of the set of features are extracted from the received medical information. Actions 410 and 420 may repeat until values for each of the features in the set of features is extracted.

When it is determined in act 420 that a value for a feature is missing or invalid, process 400 may proceed to act 430, where the missing or invalid value is modified. In some embodiments, when the value is determined to be invalid or missing, the value may be replaced with a value that may be determined in any suitable manner. For example, the replacement value may be an average value for the feature for a plurality of other patients. In some embodiments, the plurality of other patients may have the same or substantially similar medical information as the patient with the invalid or missing value. For example, the plurality of other patients may have substantially similar demographic information, surgery, medical device usage or any other suitable medical information as the patient, or any combination thereof. In some embodiments, when the value is determined to be invalid or missing, the value may be modified to include an indication that the cardiogenic shock severity information may not be able to be determined due to the invalid or missing value. For example, the indication may be implemented as a recommendation to perform one or more tests on the patient to determine the invalid or missing value. In some embodiments, the indication may be associated with a level of confidence of the cardiogenic shock severity information when the invalid or missing value is used. For example, in some embodiments, if the invalid or missing value is for a feature that may have little effect on the determined cardiogenic shock severity information, then the indication may show that the cardiogenic shock severity information was determined using the invalid or missing value but the level of confidence may be similar to that of determined cardiogenic shock severity information with all valid values. In some embodiments, the level of confidence may be associated with a color or other indicator to represent how a confidence level or score associated with the cardiogenic shock severity information.

In embodiments, where at least one of the values is determined to be invalid, the invalid value may be used to determine the cardiogenic shock severity information and the output indication may indicate the determined cardiogenic shock severity information may not be reliable and/or accurate (e.g., a low level of confidence). In some embodiments, when weighted values are used to determine the cardiogenic shock severity information, the weight associated with the invalid value may be modified (e.g., lowered) to account for the invalid value, which may cause the value of the feature to have less of an effect on the cardiogenic shock severity information determination than if the invalid value was successfully extracted and determined to be valid.

FIG. 5 shows a flowchart of process 500 for simulating changes in cardiogenic shock severity for a patient based on user input, in accordance with some embodiments of the present disclosure. In act 510, user input is received via a user interface (e.g., the user interface 230) to change one or more values used to determine cardiogenic shock severity information. For example, the user input may specify one or more values for medical information and/or device information associated with the patient. In some embodiments, the device information may include, but is not limited to, pump speed of the medical device, pressure values associated with the medical device, or any other suitable operating parameter of the medical device or a combination thereof. In some embodiments, the electronic medical information may include, but is not limited to, blood pressure, blood oxygenation saturation, heart rate, any other suitable patient health information or any combination thereof.

Process 500 may then proceed to act 520, where a simulated cardiogenic shock assessment is performed based on the user input. For example, the simulated cardiogenic shock assessment may be configured to predict cardiogenic shock severity information for the patient when values for the one or more features are changed. The simulated cardiogenic shock severity information may be used by the healthcare provider for any suitable purpose. For example, in some embodiments, the simulated cardiogenic shock severity information may be used to determine operating conditions for the medical device, which may result in a better patient outcome. For example, the simulated cardiogenic shock severity information may show that the cardiogenic shock severity of the patient may not be substantially negatively affected if the pump speed for the medical device is lowered and the healthcare provider may begin to wean the patient off of support from the medical device. In some embodiments, the simulated cardiogenic shock severity information may be used to determine that using an additional medical device may benefit the patient by reducing the risk that that the patient will transition into a more severe state of cardiogenic shock. For example, the simulated cardiogenic shock severity information may suggest that use of a pharmaceutical agent, an additional medical device, and/or change of operation of a medical device currently being used by the patient, to lower the heart rate of the patient may result in a reduced risk of severe cardiogenic shock for the patient.

Process 500 may then proceed to act 530, where an indication of the simulated cardiogenic shock severity information is displayed. In some embodiments, the indication may be output on the user interface, a wireless device (e.g., a mobile device, a tablet, a nurse's monitor) associated with the patient, a database configured to store patient health information (e.g., the medical information system 240) or any combination thereof. In some embodiments, the indication may include a tag to show that the indication is associated with the simulated cardiogenic shock severity information.

As described herein, for embodiments of the present disclosure that employ models or algorithms in which extracted features may be weighted to determine the patient risk score, the models/algorithms may be updated (e.g., retrained or tuned). For example, the models/algorithms may be updated based on whether an assessment of a healthcare professional reviewing the cardiogenic shock severity information for a patient agrees or disagrees with the output of the model/algorithm.

Having thus described several aspects and embodiments of the technology set forth in the disclosure, it is to be appreciated that various alterations, modifications, and improvements will readily occur to those skilled in the art. Such alterations, modifications, and improvements are intended to be within the spirit and scope of the technology described herein. For example, those of ordinary skill in the art will readily envision a variety of other means and/or structures for performing the function and/or obtaining the results and/or one or more of the advantages described herein, and each of such variations and/or modifications is deemed to be within the scope of the embodiments described herein. Those skilled in the art will recognize, or be able to ascertain using no more than routine experimentation, many equivalents to the specific embodiments described herein. It is, therefore, to be understood that the foregoing embodiments are presented by way of example only and that, within the scope of the appended claims and equivalents thereto, inventive embodiments may be practiced otherwise than as specifically described. In addition, any combination of two or more features, systems, articles, materials, kits, and/or methods described herein, if such features, systems, articles, materials, kits, and/or methods are not mutually inconsistent, is included within the scope of the present disclosure.

The above-described embodiments can be implemented in any of numerous ways. One or more aspects and embodiments of the present disclosure involving the performance of processes or methods may utilize program instructions executable by a device (e.g., a computer, a controller, a processor, or other device) to perform, or control performance of, the processes or methods. In this respect, various inventive concepts may be embodied as a computer readable storage medium (or multiple computer readable storage media) (e.g., a computer memory, one or more floppy discs, compact discs, optical discs, magnetic tapes, flash memories, circuit configurations in Field Programmable Gate Arrays or other semiconductor devices, or other tangible computer storage medium) encoded with one or more programs that, when executed on one or more computers or controllers or other processors, perform methods that implement one or more of the various embodiments described above. The computer readable medium or media can be transportable, such that the program or programs stored thereon can be loaded onto one or more different computers or controllers or other processors to implement various ones of the aspects described above. In some embodiments, computer readable media may be non-transitory media.

The above-described embodiments of the present technology can be implemented in any of numerous ways. For example, the embodiments may be implemented using hardware, software or a combination thereof. When implemented in software, the software code can be executed on any suitable controller or processor or collection of processors, whether provided in a single computer or distributed among multiple computers. It should be appreciated that any component or collection of components that perform the functions described above can be generically considered as a controller that controls the above-described function. A controller can be implemented in numerous ways, such as with dedicated hardware, or with general purpose hardware (e.g., one or more processor or one or more controllers) that is programmed using microcode or software to perform the functions recited above, and may be implemented in a combination of ways when the controller corresponds to multiple components of a system.

Further, it should be appreciated that a computer may be embodied in any of a number of forms, such as a rack-mounted computer, a desktop computer, a laptop computer, or a tablet computer, as non-limiting examples. Additionally, a computer may be embedded in a device not generally regarded as a computer but with suitable processing capabilities, including a Personal Digital Assistant (PDA), a smartphone or any other suitable portable or fixed electronic device.

Also, a computer may have one or more input and output devices. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computer may receive input information through speech recognition or in other audible formats.

Such computers may be interconnected by one or more networks in any suitable form, including a local area network or a wide area network, such as an enterprise network, and intelligent network (IN) or the Internet. Such networks may be based on any suitable technology and may operate according to any suitable protocol and may include wireless networks, wired networks or fiber optic networks.

Also, as described, some aspects may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

Also, the phraseology and terminology used herein is for the purpose of description and should not be regarded as limiting. The use of "including," "comprising," or "having,", "containing," "involving," and variations thereof herein, is meant to encompass the items listed thereafter and equivalents thereof as well as additional items.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

### EXEMPLARY EMBODIMENTS

A1. A method of determining cardiogenic shock information for a patient using a medical device, the method comprising: receiving, from an electronic medical record associated with a patient, medical information for the patient; extracting values for a set of features associated with cardiogenic shock from the received medical information; receiving device information associated with operation of the medical device; determining based, at least in part, on the values for the set of features and the device information, cardiogenic shock information for the patient, wherein the cardiogenic shock information includes a severity of cardiogenic shock for the patient; and outputting on a user interface associated with the medical device, an indication of the cardiogenic shock information.
A2. The method of paragraph A1, wherein the medical information for the patient includes one or more of an electronic health record, patient physiological information, patient laboratory data, and physician notes.
A3. The method of paragraph A1or A2, wherein the medical device is at least one of a mechanical circulatory support device or an extracorporeal membrane oxygenation (ECMO) machine.
A4. The method of any one of the preceding paragraphs A1 to A3, wherein the device information associated with operation of the medical device includes one or more of medical device information for the medical device, operating conditions of the medical device, or medical device flow rate.
A5. The method of any one of the preceding paragraphs A1 to A4, wherein the set of features includes one or more of blood pressure, blood flow, lactate level, blood pH level, or medication rate.
A6. The method of any one of the preceding paragraphs A1 to A5, wherein determining cardiogenic shock information for the patient comprises providing the values of the set of features and the device information as input to a trained machine learning model configured to determine the cardiogenic shock information.
A7. The method of any one of the preceding paragraphs A1 to A6, wherein determining cardiogenic shock information for the patient comprises determining a classification of severity of cardiogenic shock for the patient.
A8. The method of paragraph A7, wherein: the classification includes an alphabetic value associated with the severity of cardiogenic shock for the patient, and outputting on a user interface an indication of the cardiogenic shock information comprises displaying the alphabetic value and/or information based on the alphabetic value.
A9. The method of paragraph A7 or A8, wherein: the classification is along a continuous scale including numerical values associated with the severity of cardiogenic shock for the patient; and outputting on the user interface an indication of the cardiogenic shock information comprises displaying a numerical value of the cardiogenic shock information and/or information based on the numerical value.
A10. The method of paragraph A9, wherein the classification is continuously determined.
A11. The method of paragraph A9 or A10, wherein the classification is determined at a set interval.
A12. The method of any one of the preceding paragraphs A1 to A11, wherein determining cardiogenic shock information for the patient further comprises: providing the values for the set of features and the device information to a decision tree architecture configured to determine the cardiogenic shock information.
A13. The method of of any one of the preceding paragraphs A1 to A12, further comprising: determining that at least one value in the set of features from the received medical information is missing or invalid.
A14. The method of paragraph A13, further comprising: determining a replacement value for the at least one feature value that is not present in the set of features or is invalid, wherein determining cardiogenic shock information is based on the set of features including the replacement value.
A15. The method of paragraph A14, wherein the value in the set of features is determined to be invalid based at least in part on the value being outside of an expected range or a time associated with the value is greater than a threshold amount of time relative to a present time.
A16. The method of paragraph A14 or A15, wherein the replacement value is an estimate of the value for the invalid or missing feature based on an average of a plurality of patients.
A17. The method of any one of the preceding paragraphs A13 to A16, further comprising: outputting an indication based on the at least one value in the set of features being missing or invalid.
A18. The method of paragraph A17, wherein outputting the indication based on the at least one value in the set of features missing or being invalid comprises displaying on a user interface a color coded indication associated with the missing or invalid value.
A19. The method of paragraph A17 or A18, wherein outputting the indication based on the at least one value in the set of features missing or being invalid comprises displaying on the user interface a recommendation to perform one or more tests on the patient based on the missing or invalid value in the set of features.
A20. The method of any one of the preceding paragraphs A13 to A19, wherein determining cardiogenic shock information for the patient comprises: determining a weighted value for each of the values in the set of features; and determining a weighted feature value for the set of features based on the weighted value and the values in the set of features, wherein determining cardiogenic shock information is based at least in part on the weighted feature values for the set of features and the device information.
A21. The method of paragraph A20, wherein: the weighted value is based, at least in part, on the whether the value is an invalid value, and the weighted value for the invalid values is less than the weighted value for valid values of the set of features.
A22. The method of of any one of the preceding paragraphs A1 to A21, further comprising: performing based on the cardiogenic shock information, categorization of the patient into a risk group of a plurality of risk groups, wherein outputting the indication of the cardiogenic shock information comprises outputting an indication of the risk group for the patient.
A23. The method of paragraph A22, wherein the risk group is associated with a future severity of cardiogenic shock for the patient.
A24. The method of paragraph A23, wherein outputting an indication of the risk group for the patient comprises displaying on a user interface a color coded indication of the risk group.
A25. The method of any one of the preceding paragraphs A1 to A24, further comprising: receiving a user input via the user interface to change one or more values associated with the device information; simulating a cardiogenic shock assessment to predict the severity of the cardiogenic shock of the patient based, at least in part, on the changed one or more values, to generate simulated cardiogenic shock information; and displaying, on the user interface, a simulated indication based at least in part on the simulated cardiogenic shock information.
A26. The method of paragraph A25, wherein: the medical device is a mechanical circulatory support device; and the one or more values associated with the device information includes one or more of a flow rate, a pump speed and a pressure signal associated with the mechanical circulatory support device. A27. The method of any one of the preceding paragraphs A1 to A26, wherein: the medical device is a mechanical circulatory support device; and the received device information includes at least one of a pump speed or a pressure signal associated with the mechanical circulatory support device.
A28. The method of any one of the preceding paragraphs A1 to A27, wherein the cardiogenic shock information indicates a cardiogenic shock stage of the patient without the medical device.
A29. The method of any one of the preceding paragraphs A1 to A27, wherein determining cardiogenic shock information for the patient comprises: determining a weighted value for each of the values in the set of features; determining a weighted feature value for the set of features based on the weighted value and the values for the set of features; and determining, based at least in part on the weighted feature values for the set of features and the device information, cardiogenic shock information.
A30. A method of predicting a future severity of cardiogenic shock for a patient using a medical device, the method comprising: receiving, from an electronic medical record associated with a patient, medical information for the patient; extracting values for a set of features associated with cardiogenic shock from the received medical information; receiving device information associated with operation of the medical device; determining based, at least in part, on the values for the set of features and the device information, cardiogenic shock information for the patient, wherein the cardiogenic shock information includes a future severity of cardiogenic shock for the patient; and outputting on a user interface associated with the medical device, an indication of the cardiogenic shock information.

## Claims

1. A system of determining cardiogenic shock information for a patient using a medical device, the system comprising:
a controller in communication with a medical information system and the medical device, the controller being configured to:
receive, from the medical information system associated with a patient, medical information for the patient;
extract values for a set of features associated with cardiogenic shock from the received medical information;
receive device information associated with operation of the medical device;
determine based, at least in part, on the values for the set of features and the device information, cardiogenic shock information for the patient, wherein the cardiogenic shock information includes a severity of cardiogenic shock for the patient; and
output on a user interface associated with the medical device, an indication of the cardiogenic shock information.

2. The system of claim 1, wherein the medical information for the patient includes one or more of an electronic health record, patient physiological information, patient laboratory data, and physician notes, and/ or
wherein the medical device is at least one of a mechanical circulatory support device or an extracorporeal membrane oxygenation (ECMO) machine, and/ or
wherein the device information associated with operation of the medical device includes one or more of medical device information for the medical device, operating conditions of the medical device, or medical device flow rate, and/ or
wherein the set of features includes one or more of blood pressure, blood flow, lactate level, blood pH level, or medication rate, and/ or
wherein determining cardiogenic shock information for the patient by the controller comprises providing the values of the set of features and the device information as input to a trained machine learning model configured to determine the cardiogenic shock information.

3. The system of claim 1 or 2, wherein determining cardiogenic shock information for the patient by the controller comprises determining a classification of severity of cardiogenic shock for the patient.

4. The system of claim 3, wherein:
the classification includes an alphabetic value associated with the severity of cardiogenic shock for the patient, and
outputting on a user interface an indication of the cardiogenic shock information by the controller comprises displaying the alphabetic value and/or information based on the alphabetic value, and/ or wherein:
the classification is along a continuous scale including numerical values associated with the severity of cardiogenic shock for the patient; and
outputting on the user interface an indication of the cardiogenic shock information by the controller comprises displaying a numerical value of the cardiogenic shock information and/or information based on the numerical value,
preferably wherein the classification is continuously determined, and/ or wherein the classification is determined at a set interval.

5. The system of any one of the preceding claims, wherein determining cardiogenic shock information for the patient by the controller further comprises:
providing the values for the set of features and the device information to a decision tree architecture configured to determine the cardiogenic shock information.

6. The system of any one of the preceding claims, wherein the controller is further configured to:
determine that at least one value in the set of features from the received medical information is missing or invalid.

7. The system of claim 6, wherein the controller is further configured to:
determine a replacement value for the at least one feature value that is not present in the set of features or is invalid, wherein determining cardiogenic shock information is based on the set of features including the replacement value,
preferably wherein the value in the set of features is determined to be invalid based at least in part on the value being outside of an expected range or a time associated with the value is greater than a threshold amount of time relative to a present time,
preferably wherein the replacement value is an estimate of the value for the invalid or missing feature based on an average of a plurality of patients.

8. The system of claim 6 or 7, wherein the controller is further configured to:
output an indication based on the at least one value in the set of features being missing or invalid,
preferably wherein outputting the indication based on the at least one value in the set of features missing or being invalid by the controller comprises displaying on a user interface a color coded indication associated with the missing or invalid value, and/ or
preferably wherein outputting the indication based on the at least one value in the set of features missing or being invalid by the controller comprises displaying on the user interface a recommendation to perform one or more tests on the patient based on the missing or invalid value in the set of features.

9. The system of any one of the preceding claims 6 to 8, wherein determining cardiogenic shock information for the patient by the controller comprises:
determining a weighted value for each of the values in the set of features; and
determining a weighted feature value for the set of features based on the weighted value and the values in the set of features, wherein determining cardiogenic shock information is based at least in part on the weighted feature values for the set of features and the device information;
the weighted value is preferably based, at least in part, on the whether the value is an invalid value, and
the weighted value for the invalid values is less than the weighted value for valid values of the set of features.

10. The system of any one of the preceding claims, wherein the controller is further configured to:
perform based on the cardiogenic shock information, categorization of the patient into a risk group of a plurality of risk groups, wherein outputting the indication of the cardiogenic shock information by the controller comprises outputting an indication of the risk group for the patient.

11. The system of claim 10, wherein the risk group is associated with a future severity of cardiogenic shock for the patient, and/ or
wherein outputting an indication of the risk group for the patient comprises displaying on a user interface a color coded indication of the risk group.

12. The system of any one of the preceding claims, wherein the controller is further configured to:
receive a user input via the user interface to change one or more values associated with the device information;
simulate a cardiogenic shock assessment to predict the severity of the cardiogenic shock of the patient based, at least in part, on the changed one or more values, to generate simulated cardiogenic shock information; and
display, on the user interface, a simulated indication based at least in part on the simulated cardiogenic shock information,
preferably wherein:
the medical device is a mechanical circulatory support device; and
the one or more values associated with the device information includes one or more of a flow rate, a pump speed and a pressure signal associated with the mechanical circulatory support device.

13. The system of any one the preceding claims, wherein:
the medical device is a mechanical circulatory support device; and
the received device information includes at least one of a pump speed or a pressure signal associated with the mechanical circulatory support device, and/ or
wherein the cardiogenic shock information indicates a cardiogenic shock stage of the patient without the medical device.

14. The system of any one of the preceding claims, wherein determining cardiogenic shock information for the patient by the controller comprises:
determining a weighted value for each of the values in the set of features;
determining a weighted feature value for the set of features based on the weighted value and the values for the set of features; and
determining, based at least in part on the weighted feature values for the set of features and the device information, cardiogenic shock information.

15. A system of predicting a future severity of cardiogenic shock for a patient using a medical device, the system comprising:
a controller in communication with a medical information system and the medical device, the controller being configured to:
receive, from the medical information system associated with a patient, medical information for the patient; extract values for a set of features associated with cardiogenic shock from the received medical information;
receive device information associated with operation of the medical device;
determining based, at least in part, on the values for the set of features and the device information, cardiogenic shock information for the patient, wherein the cardiogenic shock information includes a future severity of cardiogenic shock for the patient; and
output on a user interface associated with the medical device, an indication of the cardiogenic shock information.
